# EUROPEAN PATENT APPLICATION

(11) **EP 4 434 531 A1**
(43) Date of publication of application: **25.09.2024**
(21) Application number: 22896083.7
(22) Date of filing: 17.11.2022
(51) Int. Cl.: A61K 36/185, A61K 36/74, A61K 36/536, A61K 36/9066, A61P 35/00, A23L 33/105

(54) **PHARMACEUTICAL COMPOSITION AND HEALTH FUNCTIONAL FOOD FOR CANCER PREVENTION OR TREATMENT, COMPRISING MIXED HERBAL MEDICINE EXTRACT**

(30) Priority: 17.11.2021 KR 20210158287
(71) Applicant: H&O Biosis Co., Ltd., Hwaseong-si Gyeonggi-do 18514 (KR)
(72) Inventor: PARK, Jong Min, Anyang-si Gyeonggi-do 14105 (KR)
(74) Representative: Longland, Emma Louise
(86) International application number: PCT/KR2022/018161
(87) International publication number: WO 2023/090881

(57) **Abstract**

The present invention relates to an anticancer pharmaceutical composition or health functional food including a mixed extract, and specifically, including a mixed extract of *Hedyotis diffusa, Prunella vulgaris, Akebia quinata, Curcuma zedoaria,* and Curcumae Radix. The mixed extract of the present invention is effective in inhibiting cancer cell proliferation and metastasis in various carcinomas, and thus can be usefully utilized as an anticancer pharmaceutical composition or as an anticancer health functional food.

## Description

### [Technical Field]

The present invention relates to an anticancer pharmaceutical composition or health functional food including a mixed extract of *Hedyotis diffusa, Prunella vulgaris, Akebia quinata, Curcuma zedoaria,* and Curcumae Radix.

### [Background Art]

For the treatment of cancer, which is one of the major health issues worldwide, therapies such as radiation therapy, chemotherapy, and surgery have been developed. Among them, chemotherapy is a drug treatment that mainly inhibits or destroys tumor cell proliferation. Despite the certain achievements in the field of anticancer to date, the chemotherapy still has serious issues such as poor clinical prognosis, high recurrence rate, high metastasis rate, and short survival period. In particular, single chemotherapeutic agent-based chemotherapy causes a major technical issue of reducing the efficacy of cancer treatment, such as the development of resistance in cancer patients.

As an alternative to chemotherapy based on a single drug, traditional Chinese medicine (TCM), an East Asian traditional medicine based on herb research, is attracting attention. According to a recent TCM review thesis on anticancer therapy, single drug prescriptions are rarely used in TCM, and a unique combination of multiple herbs is prescribed according to TCM medical theory and individual physical constitution (Fan, Yuqi, et al. "Anti-tumor activities and mechanisms of Traditional Chinese medicines formulas: A review." Biomedicine & Pharmacotherapy 132 (2020): 110820.). These prescriptions target multiple targets for cancer treatment and are intended to act in a complex way on multiple signaling pathways. As a result, they can be used as excellent alternative anticancer therapies with high efficacy and low side effects compared to single drugs. However, in general, when single drugs are combined, the effects of each drug may be offset from each other or one another, so a simple mixture of two or more drugs does not achieve the desired therapeutic effect. Up to now, combinations of herbs discovered based on TCM theory remain limited.

### [Disclosure]

### [Technical Problem]

In the present invention, the technical problem to be solved is to provide a method for preventing or treating cancer through a mixed medicinal extract.

### [Technical Solution]

The present inventors have discovered a unique combination of herbs that have not been tried or adopted thus far and identified that the combination thereof may be usefully utilized in anticancer therapy. Specifically, the present invention proposes that a composition configured of a mixed extract of *Hedyotis diffusa, Prunella vulgaris, Akebia quinata, Curcuma zedoaria,* and Curcumae Radix may be usefully utilized for the prevention or treatment of cancer.

Unless otherwise defined, terms used herein are used in the same context as generally adopted in the field of botany and oriental herbal medicine. In the present invention, oriental herbal medicine is a traditional medicine utilized in East Asia such as China, Korea, and Japan, and unless otherwise stated, oriental herbal medicine is used in the context of covering Chinese medicine, Korean medicine, and Kampo medicine and so on.

The present invention provides an anticancer composition including an extract of *Hedyotis diffusa, Prunella vulgaris, Akebia quinata, Curcuma zedoaria,* and Curcumae Radix.

*Hedyotis diffusa* is a plant belonging to the family Rubiaceae, and the *Hedyotis diffusa* used in a mixed extract of the present invention may be a whole plant of *Hedyotis diffusa.* The stalk of *Hedyotis diffusa* is thin and coiled like a vine, and the outer side is greenish gray or grayish brown. The ovules are fragile and easily broken. The broken side has a white stamen in the center. The leaves are mostly broken, severely wrinkled, and fall off easily. Flowers are small and white, and there is one hanging on the leaf axil, but sometimes two. The root is not branched and has a diameter of 2-4 mm and has beard roots.

*Prunella vulgaris* is a plant belonging to the family Lamiaceae, and the *Prunella vulgaris* used in a mixed extract of the present invention may be a spike of *Prunella vulgaris.* The spike of *Prunella vulgaris* is attached with many bracts and calyxes, is close to a columnar shape, and is 3-6 cm long and 10-15 mm in diameter. The outer side is grayish brown or reddish brown, and the ovules are light. The corolla remains at the top, and the stem at the bottom, and there are 4 loments in the calyx. The bracts are heart-shaped or eccentric, and the calyx and venation have white hairs. This medicine has little to no odor and taste.

*Akebia quinata* is a plant belonging to the family Lardizabalaceae, and the *Akebia quinata* used in a mixed extract of the present invention may be a fruit of *Akebia quinata.* The fruit of *Akebia quinata* is oval, slightly curved, 3-5 cm long, and 25-35 mm in diameter. The outer side is grayish brown or dark brown. The fruit skin is crumpled and there are irregular net-shaped apophyses. There are marks of the stigma at the end and round marks where the fruit skins have fallen off at the bottom. The ovules are hard and the cut side is light yellow and contains many black seeds.

*Curcuma zedoaria* is a plant belonging to the family Zingiberaceae, and the *Curcuma zedoaria* used in a mixed extract of the present invention may be a rhizome of *Curcuma zedoaria.* The rhizome of *Curcuma zedoaria* is ovate and 25-40 mm in diameter and 4-6 cm long. The outer side is grayish light brown or grayish brown. The nodes are conspicuous in an annular shape, 5-8 mm between the nodes. There are thin vertical wrinkles, traces of removing roots, and small apophyses on the lateral rhizomes. When looking through a magnifying glass, there are rough hairs on the outer side of which are cutin and thus are hard to cut. The transverse section is grayish brown, and a light grayish brown line is visible between the 2-5 mm thick cortex and the wide central cylinder.

Curcumae Radix is a plant belonging to the family Zingiberaceae, and in the present invention, Curcumae Radix is defined as the root of a plant of the genus Curcuma. Curcumae Radix used in a mixed extract of the present invention may be a tuberous root of a plant of the genus Curcuma.

Specifically, Curcumae Radix of the present invention may be a tuberous root of one or more plants selected from the group consisting of *Curcuma wenyujin, Curcuma longa, Curcuma kwangsiensis, Curcuma phaeocaulis, and Curcuma aromatica.*

Among them, the tuberous root of *Curcuma wenyujin* is long round or oval, 35-70 mm long and 12-25 mm in diameter. The outer side is grayish brown, and there are uneven vertical wrinkles, and the spot where the vertical wrinkles protrude is relatively light in color. The ovules are hard and the cut side is grayish brown, keratinous, and the annular pattern of endothelial layer is clear. It has a characteristic smell and the taste is slightly bitter.

In addition, the tuberous root of *Curcuma longa* is fusiform, 25-45 mm long and 10-15 mm in diameter, and there are some that are long and thin on one side. The outer side is grayish brown or grayish yellow with vertical wrinkles. The cross section is orange and the outer side is yellowish brown or reddish brown. It has a characteristic smell and the taste is very spicy.

In addition, the tuberous root of *Curcuma kwangsiensis* is long conical or long round, 20-65 mm long and 10-18 mm in diameter. The outer side has shallow vertical wrinkles or coarse reticulated wrinkles. It has a characteristic smell and the taste is slightly spicy and bitter.

In addition, the tuberous root of *Curcumaphaeocaulis* is long oval, 15-35 mm long and 10-12 mm in diameter, and relatively thick and large. It has a characteristic smell and the taste is mild.

It is preferable that the mixed extract of the present invention is mixed with 40 to 150 parts by weight of *Prunella vulgaris,* 30 to 120 parts by weight of *Akebia quinata,* 20 to 80 parts by weight of *Curcuma zedoaria,* and 20 to 80 parts by weight of Curcumae Radix with respect to 100 parts by weight of *Hedyotis diffusa.* For a more preferred mixture ratio, reference may be made to Preparation Examples to be described later.

The extract used herein refers to an agent prepared by squeezing herb into a suitable leaching solution, and concentrating the leaching solution through evaporation, and may refer to a liquid extract obtained by extraction, a diluent or concentrate of the liquid extract, a dry product obtained by drying the liquid extract, or a crude purified product or a purified product. The mixed extract of the present invention may be prepared using a general extraction method, separation, and purification method known in the field of natural products. As an extraction method of the mixed extract of the present invention, methods such as boiling extraction, hot water extraction, cold-immersion extraction, reflux cooling extraction, or ultrasonic extraction may be used, and in addition, natural products or known methods in the field of oriental herbal medicine may be used.

In the present invention, the mixed extract may be prepared through extraction with an extraction solvent or through fractionation by applying a fractionation solvent to an extract prepared by extraction with an extraction solvent. The extraction solvent of the present invention may be water, an organic solvent, or a mixed solvent thereof, and the organic solvent may be a polar solvent such as an alcohol having 1 to 4 carbon atoms, ethyl acetate, or acetone, a non-polar solvent such as hexane or dichloromethane, or a mixed solvent thereof. Preferably, water, an alcohol having 1 to 4 carbon atoms, or a mixed solvent thereof may be used as the extraction solvent.

In one embodiment, the mixed extract of the present invention may be prepared by a preparation method including the following steps.
(1) removing and washing foreign substances mixed in each herbal medicine;
(2) extracting an extract by adding an extraction solvent to the washed herbal mixture;
(3) filtering the extract;
(4) concentrating the filtered extract under reduced pressure;
(5) filtering concentrate; and
(6) freeze-drying the filtered concentrate.

It is preferable that the washing of step (1) is repeated 1 to 5 times.

The extraction of step (2) may use a shaking extraction, Soxhlet extraction, or reflux extraction method, but is not limited thereto.

As the extraction solvent, water, an alcohol having 1 to 4 carbon atoms, or a mixture thereof may be used, and hot water extraction is most preferable. It is preferable that the extraction solvent is extracted by adding an extraction solvent 5 to 20 times the total weight of the prepared herbal mixture.

The hot water pressure extraction temperature is preferably 60 to 100°C, and more preferably 80 to 100°C. In addition, the extraction time is preferably 1 to 24 hours, and the number of extractions is preferably 1 to 5 times.

The filtration of the extract of step (3) is to separate solid particles from a fluid by passing the same through a filtering medium so that particles larger than micropores are deposited on a membrane. For example, the extract prepared in step (2) may be filtered through conventional filtration.

It is preferable that the concentration under reduced pressure in step (4) is performed using a vacuum decompression concentrator or a vacuum rotary evaporator. In step (4), it is preferable to concentrate the liquid extract so as to have a solid content of 40% or more.

The filtration of the concentrate of step (5) may be performed in the same manner as the filtration of the extract of step (3) described above, and a gravity filter, a vacuum filter, a pressure filter, a compression filter, a centrifugal filter, etc. may be used, but is not limited thereto.

The drying of step (6) may use a reduced pressure drying, vacuum drying, boiling drying, spray drying, or freeze-drying method, but is not limited thereto.

A composition including extracts of *Hedyotis diffusa, Prunella vulgaris, Akebia quinata, Curcuma zedoaria,* and Curcumae Radix according to an embodiment of the present invention is excellent in preventing or treating cancer.

The present inventors identified the excellent anticancer efficacy in a mixed extract of *Hedyotis diffusa, Akebia quinata, Prunella vulgaris, Curcuma zedoaria,* and Curcumae Radix through experiments. Specifically, in the present invention, a mixed extract prepared by mixing and then extracting medicine in a ratio of 40 to 150 parts by weight of *Prunella vulgaris,* 30 to 120 parts by weight of *Akebia quinata, 20* to 80 parts by weight of *Curcuma zedoaria,* and 20 to 80 parts by weight of Curcumae Radix with respect to 100 parts by weight of *Hedyotis diffusa* was identified to produce an effect of inhibiting the proliferation of cancer cells or killing cancer cells of various carcinomas such as liver cancer, lung cancer, stomach cancer, colorectal cancer, breast cancer, melanoma, and pancreatic cancer (Example 1; FIGS. 1A to 1C), an effect of inhibiting apoptosis, which is the pathogenesis of cancer, or the effect of inhibiting the expression of cancer-related proteins (Example 2; FIGS. 2A to 2C), an synergistic anticancer effect when combined with the anticancer drug sorafenib (Example 3; FIG. 3), an anticancer effect on anticancer drug-resistant cancer (Example 4; FIGS. 4A to 4C), and an inhibitory effect on metastatic cancer (Example 5; FIG. 5). Moreover, it was identified that the cell cycle of cancer cells was induced into the Sub-G1 cycle (Example 6; FIGS. 6A to 6F), and the accumulation of reactive oxygen species was increased (Example 7; FIGS. 7A to 7D). In addition, the mixed extract of the present invention induced proliferation of M1 macrophages, which are immune cells (Example 8; FIGS. 8A and 8B), and induced cytokine production of cancer cells and immune cells (Example 9; FIGS. 9A to 9F), thereby identifying the anticancer effect by the regulation of the immune system. In addition, it was identified that it effectively inhibited angiogenesis required for cancer cell proliferation (Example 10; FIG. 10). Accordingly, the mixed extract including *Hedyotis diffusa, Akebia quinata, Prunella vulgaris, Curcuma zedoaria,* and Curcumae Radix may be usefully utilized as an anticancer pharmaceutical composition or as an anticancer health functional food.

As used herein, the term "cancer" refers to a lump that has abnormally grown due to autonomous overgrowth of the body's tissue, and may be divided into benign and malignant tumors. While benign tumors have a relatively slow growth rate and do not metastasize, malignant tumors infiltrate into surrounding tissues, grow rapidly, and spread or metastasize to various parts of the body, thus endangering life. Accordingly, malignant tumors may be regarded as the same meaning as cancer.

Cancer that may be prevented, improved, or treated by the mixed extract of the present invention is not limited to a specific cancer, preferably breast cancer, lung cancer, stomach cancer, liver cancer, blood cancer, bone cancer, pancreatic cancer, melanoma, skin cancer, head or neck cancer, skin or eye melanoma, uterine sarcoma, ovarian cancer, rectal cancer, anal cancer, colorectal cancer, fallopian tubal cancer, endometrial cancer, cervical cancer, small intestine cancer, endocrine cancer, thyroid cancer, parathyroid cancer, kidney cancer, soft tissue tumor, urethral cancer, prostate cancer, bronchial cancer, or bone marrow cancer. More preferably, the cancer may be liver cancer, lung cancer, stomach cancer, colorectal cancer, pancreatic cancer, or melanoma, but is not limited thereto.

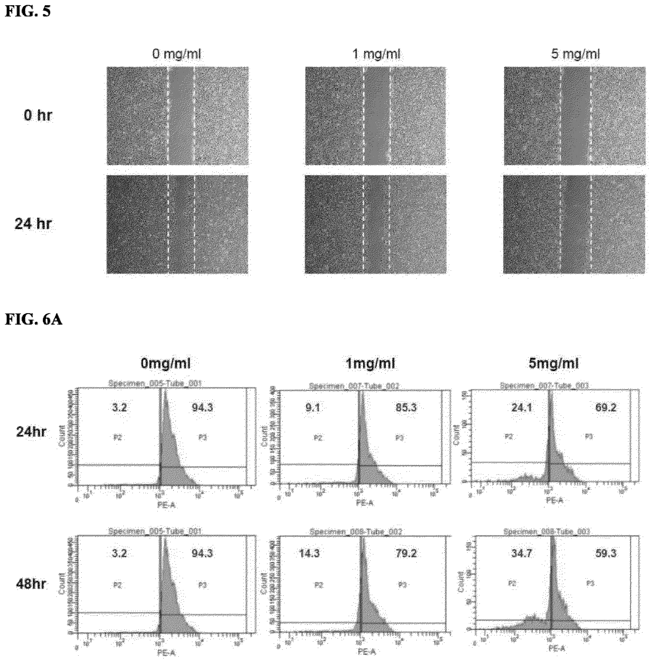

In the present invention, the cancer may be metastatic cancer. In the present invention, it was identified that the mixed extract may effectively inhibit the metastasis of cancer cells (Example 5; FIG. 5). Accordingly, the mixed extract according to the present invention may be usefully utilized for the prevention or treatment of metastatic cancer.

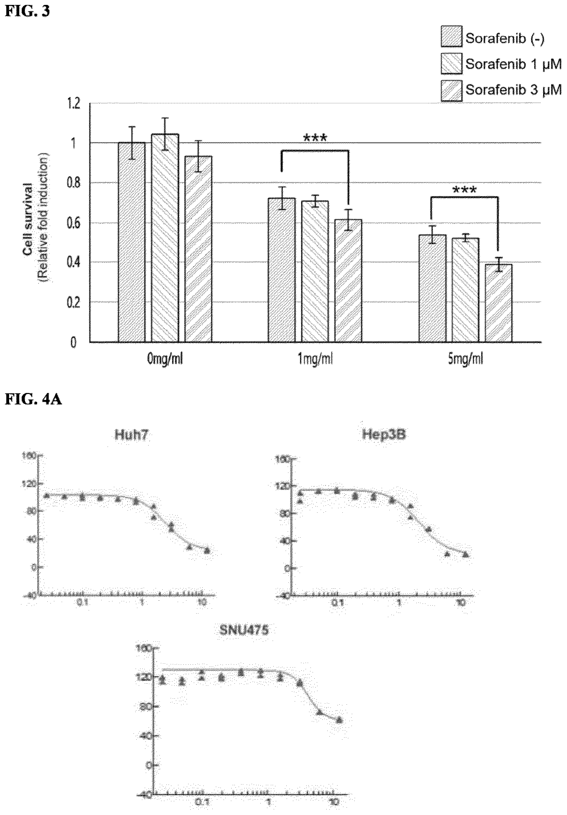

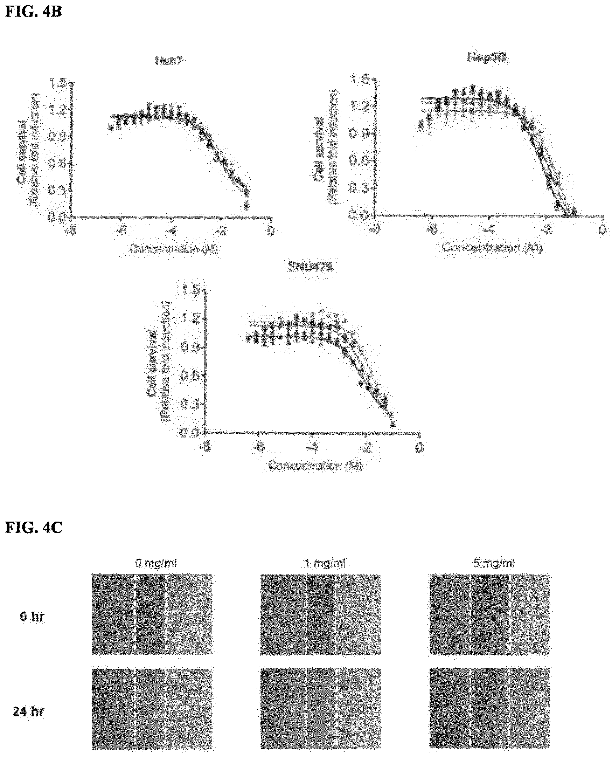

In the present invention, cancer may be a cancer having resistance to an anticancer drug. Anticancer drug resistance refers to a case in which cancer cells do not die despite administration of an amount of an anticancer drug that may reach a blood concentration capable of killing cancer cells, and it has been pointed out as one of the obstacles to cancer treatment using the conventional chemotherapy. In the present invention, in the SNU475 cell line (FIG. 4A), which is known to have drug resistance to sorafenib or regorafenib, it was identified that cancer cell death and cancer metastasis inhibition were effectively induced during the treatment of the mixed extract according to the present invention (Example 4; FIGS. 4B and 4C). Accordingly, the mixed extract according to the present invention may be usefully utilized for the prevention or treatment of anticancer drug-resistant cancer, specifically, tyrosine kinase inhibitor-resistant cancer such as sorafenib or regorafenib.

The mixed extract according to the present invention may be used in combination with one or more anticancer drugs to improve anticancer therapy. The anticancer drug used in combination may be one or more known anticancer drugs, for example, it may be a targeted anticancer drug, but is not limited thereto. In the present invention, when the standard anticancer drug sorafenib and the mixed extract of the present invention were used in combination, it was identified that the anticancer effect was increased compared to each single therapy (Example 3; FIG. 3). Accordingly, the mixed extract according to the present invention may be usefully utilized not only as a single therapy but also as a combination therapy with a conventional anticancer drug.

The mixed extract including *Hedyotis diffusa, Akebia quinata, Prunella vulgaris, Curcuma zedoaria,* and Curcumae Radix according to the present invention may further include a portion of one or more herbal extracts to exert the anticancer efficacy identified in the present invention, maximize the efficacy of the herbal extract according to the principles of oriental herbal medicine, reduce side effects, improve bioavailability, and improve the convenience of taking medicine. However, the anticancer effect of the herbal extract of the present invention is produced due to the synergistic effect of mixing the extracts of *Hedyotis diffusa, Akebia quinata, Prunella vulgaris, Curcuma zedoaria,* and Curcumae Radix. An aspect of the invention of providing an anticancer use of a herbal extract is achieved even when one or more of the above-listed herbal extracts, or one or more other known herbal extracts are not included.

Thus, the composition including the mixed extract according to the present invention may exhibit anticancer effects through the activity of the inhibition of metastasis of cancer cells, the induction of apoptosis, or the inhibition of cell growth.

As used herein, the term "prevention" means all actions for inhibiting or delaying a disease through the administration of the composition including the mixed extract. In addition, as used herein, the term "treatment" means all actions by which symptoms of a disease are relieved or take a turn for the better through administration of the composition including the mixed extract.

The present invention also provides an anticancer pharmaceutical composition including the mixed extract as an active ingredient.

In the present invention, the pharmaceutical composition may be used by adding the mixed extract of the present invention or together with one or more herbal extracts and/or anticancer drugs or additives, and may be appropriately used according to a conventional method. In addition, the pharmaceutical composition of the present invention may prevent or treat cancer by inhibiting the proliferation and metastasis of cancer cells in various carcinomas.

The pharmaceutical composition of the present invention may be administered in various forms of preparation such as oral and parenteral administration in case of actual clinical administration. Common diluents or excipients such as filler, extender, binder, humectants, disintegrant, and surfactant may be used for formulation thereof.

Solid preparations for oral administration include tablets, pills, powders, granules, capsules, and the like, and these solid preparations may be manufactured by mixing at least one or more excipients such as starch, calcium carbonate, sucrose, lactose, and gelatin, with the pharmaceutical composition of the present invention. In addition, lubricants such as magnesium, stearate, and talc may be used as well as simple excipients.

Liquid preparations for oral administration include suspensions, liquid for internal use, emulsions, syrups, etc. but various excipients such as humectants, sweetening agents, aromatic agents, and preservatives may be included as well as simple diluents such as water and liquid paraffin.

Preparations for parenteral administration include sterile aqueous solution, nonaqueous solvent, suspensions, emulsions, lyophilizer, and suppository. Propylene glycol, polyethylene, glycol, vegetable oils such as olive oil, and injectable esters such as ethyl oleate may be used as the nonaqueous solvent and the suspensions. Base of the suppository may be witepsol, macrogol, tween 61, cacao butter, laurinum, glycerol, gelatin, etc. The pharmaceutical composition of an embedment of the present invention may be administered by subcutaneous injection, intravenous injection, or intramuscular injection when applying parenteral administration.

Dosage of the pharmaceutical composition of the present invention varies by condition and weight of a patient, degree of disease, form of drug, route of administration, and duration of administration. It may be selected by those skilled in the art pro re nata. However, administering 0.001-3,000 mpk (mg/kg) of the extract of the present invention per day is preferable to achieve desired effects, and more specifically, 0.01 to 1,000 mpk per day.

Administration may be once a day or divided into several times per day. However, the said dosage explained hereinbefore does not limit the scope of the present invention.

The pharmaceutical composition of the present invention may be used in combination with conventional anticancer therapy for anticancer therapy.

In another aspect, the present invention provides a method for preventing or treating cancer, in which the method includes administering a mixed extract or a composition including the same to a subject in need thereof.

As used herein, the term "subject" refers to all animals including humans that have already developed cancer or are at risk of developing cancer, and the disease may be effectively prevented and treated by administering the mixed extract and the composition including the same of the present invention to a subject.

The present invention also provides an anticancer health functional food including the mixed extract of the present invention as an active ingredient. In the present invention, the health functional food refers to a food prepared using raw materials or ingredients having useful functionality to the human body. The term "health functional food" is interchangeable with terms (for example, functional food, etc.) generally adopted and used in the country where the present application is applied as long as the above definition is satisfied.

In the present invention, the health functional food may be used with the mixed extract of the present invention or used together with one or more other food ingredients and/or food additives, and may be appropriately used according to a conventional method.

In the present invention, the suitability of food additives may be determined according to the standards approved by the regulatory authorities of the country where the present application is applied, for example, chemical synthetic products such as ketones, glycine, potassium citrate, nicotinic acid, and cinnamic acid; natural additives such as persimmon dye, licorice extract, crystalline cellulose, kaoliang color, and guar gum; and mixed formulations such as L-glutamine sodium formulations, alkali agents for noodles, preservative formulations, and tar color formulations.

In the health functional food of the present invention, the content of the mixed extract may be appropriately decided according to the purpose of use (prevention, health, or therapeutic treatment). In general, in the preparation of food or beverage, the mixed extract of the present invention may be added in an amount of 0.01 to 15% by weight based on the total weight of the food.

There is no particular limitation on the type of health functional food in the present invention. Examples of health functional foods to which the mixed extract of the present invention may be added include beverages, gums, vitamin complexes, drinks, and the like, and include all health functional foods as a general meaning. As the health functional food, an easy-to-take health functional beverage is the most preferable.

The health functional beverage of the present invention may contain various flavoring agents or natural carbohydrates as additional ingredients, like conventional beverages. Examples of the natural carbohydrate include monosaccharides such as glucose, and fructose; disaccharides such as maltose, and sucrose; and polysaccharides such as conventional sugars such as dextrin, and cyclodextrin, and sugar alcohols such as xylitol, sorbitol, and erythritol. In addition, flavoring agents other than those described above (saccharin, aspartame, etc.) may be used.

The health functional beverage of the present invention may contain various nutrients, vitamins, minerals (electrolytes), flavors such as synthetic flavorings and natural flavorings, colorants and enhancers (cheese, chocolate, and the like), pectic acid and salts thereof, alginic acid and salts thereof, organic acids, protective colloid thickeners, pH adjusting agents, stabilizers, preservatives, glycerin, alcohols, carbonating agents used in carbonated beverages, and the like.

The present invention also provides a method for preventing or treating cancer, in which the method includes administering to a patient an extract of *Hedyotis diffusa, Prunella vulgaris, Akebia quinata, Curcuma zedoaria,* and Curcumae Radix.

The present invention also provides a method for preventing or alleviating cancer, in which the method includes ingesting an extract of *Hedyotis diffusa, Prunella vulgaris, Akebia quinata, Curcuma zedoaria,* and Curcumae Radix into humans.

The present invention also provides a use of an extract of *Hedyotis diffusa, Prunella vulgaris, Akebia quinata, Curcuma zedoaria,* and Curcumae Radix for the preparation of anticancer drugs.

The present invention also provides a use of an extract of *Hedyotis diffusa, Prunella vulgaris, Akebia quinata, Curcuma zedoaria,* and Curcumae Radix for the preparation of an anticancer health functional food.

### [Advantageous Effects]

The mixed extract of *Hedyotis diffusa, Prunella vulgaris, Akebia quinata, Curcuma zedoaria,* and Curcumae Radix according to the present invention can be effectively utilized for the prevention or treatment of cancer. Accordingly, the mixed extract of the present invention can be usefully utilized as an anticancer pharmaceutical composition or as an anticancer health functional food.

### [Description of Drawings]

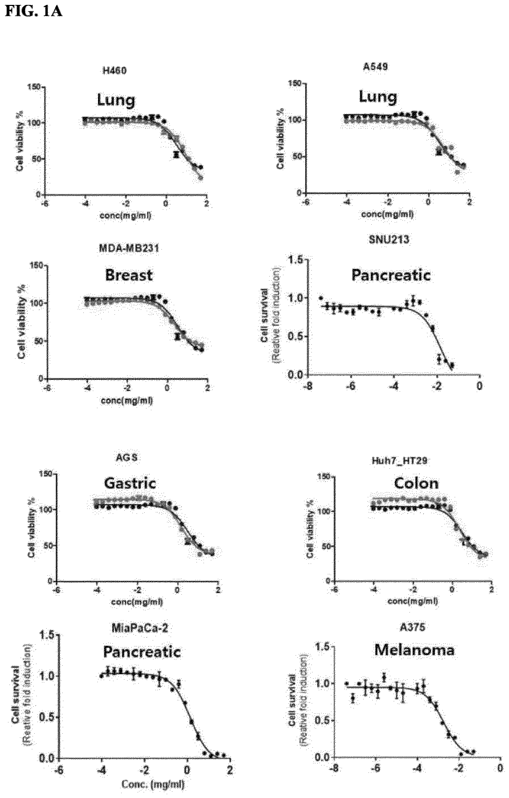

FIGS. 1A to 1C show the effect of the mixed extract of the present invention on apoptosis of cancer cells.

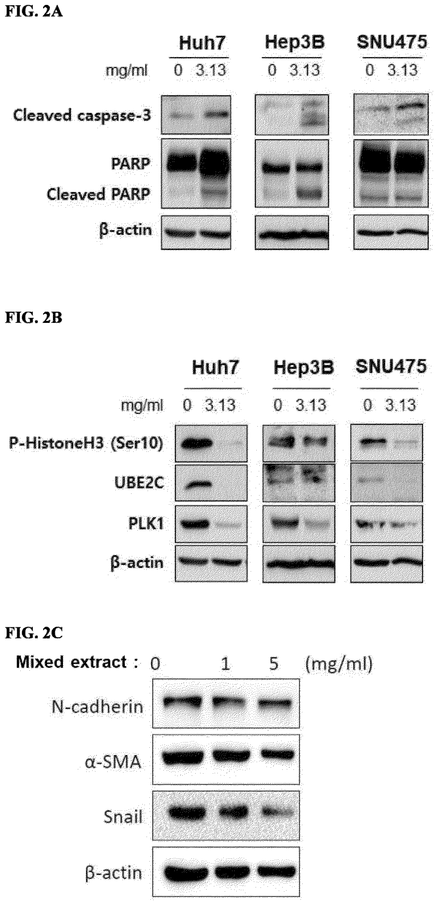

FIGS. 2A to 2C show the effect of the mixed extract of the present invention on the expression of proteins involved in cancer cells.
FIG. 3 shows the effect of the combined use of the mixed extract of the present invention and the anticancer drug sorafenib on apoptosis of cancer cells.
FIGS. 4A to 4C show the effect of the mixed extract of the present invention on apoptosis and metastasis of cancer cells resistant to the anticancer drug sorafenib.
FIG. 5 shows the effect of the mixed extract of the present invention on metastasis of cancer cells.

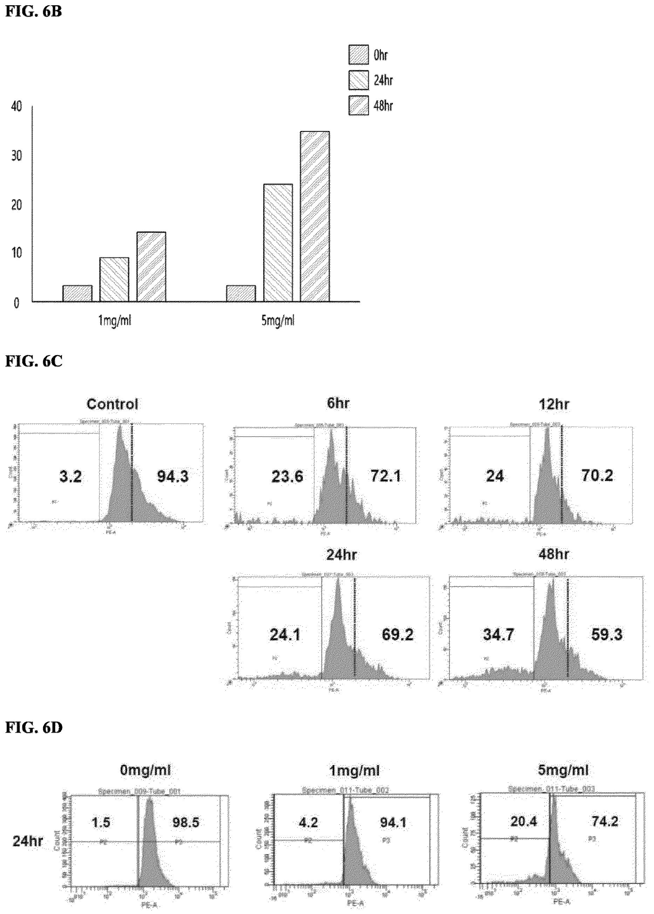

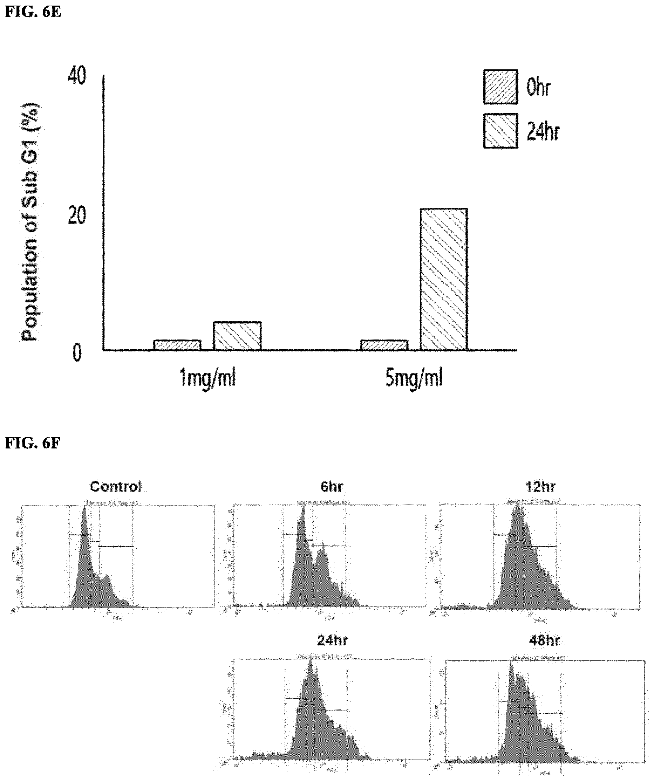

FIGS. 6A to 6F show the effect of the mixed extract of the present invention on a cancer cell cycle.

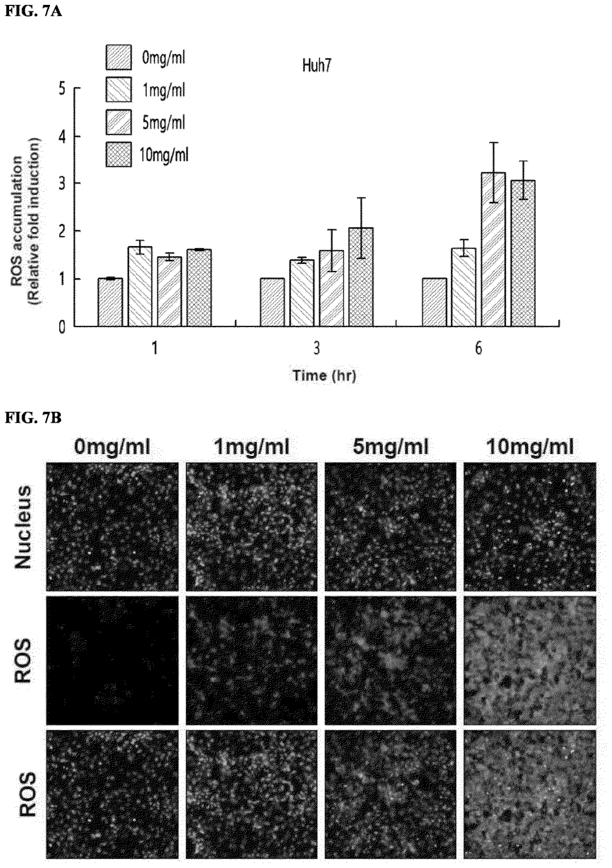

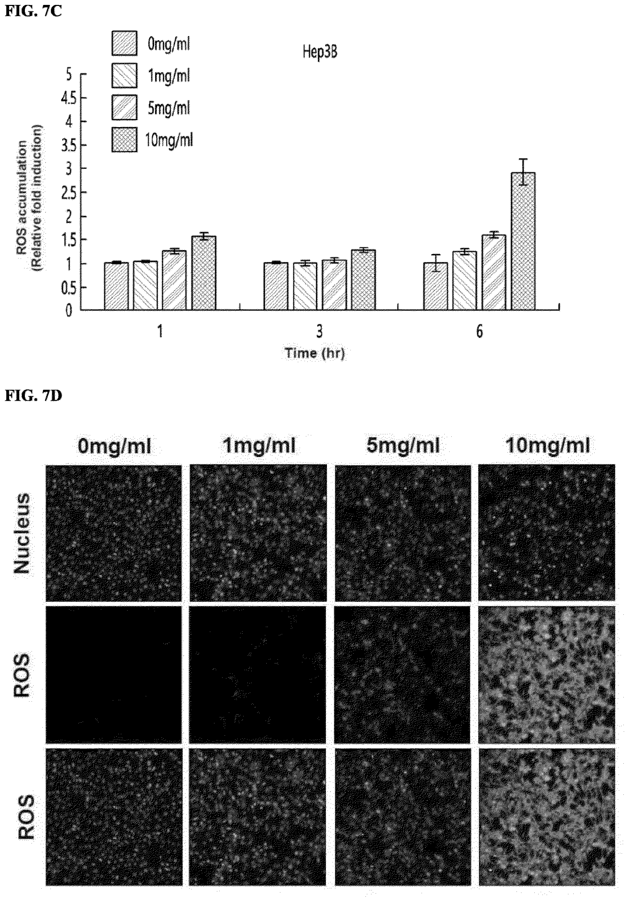

FIGS. 7A to 7D show the effect of the mixed extract of the present invention on the accumulation of reactive oxygen species in cancer cells.

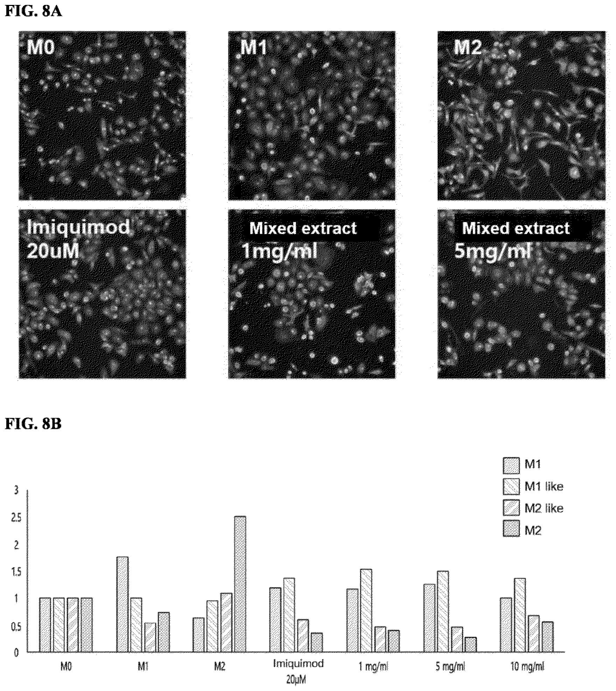

FIGS. 8A and 8B show the effect of the mixed extract of the present invention on the proliferation of immune cells.

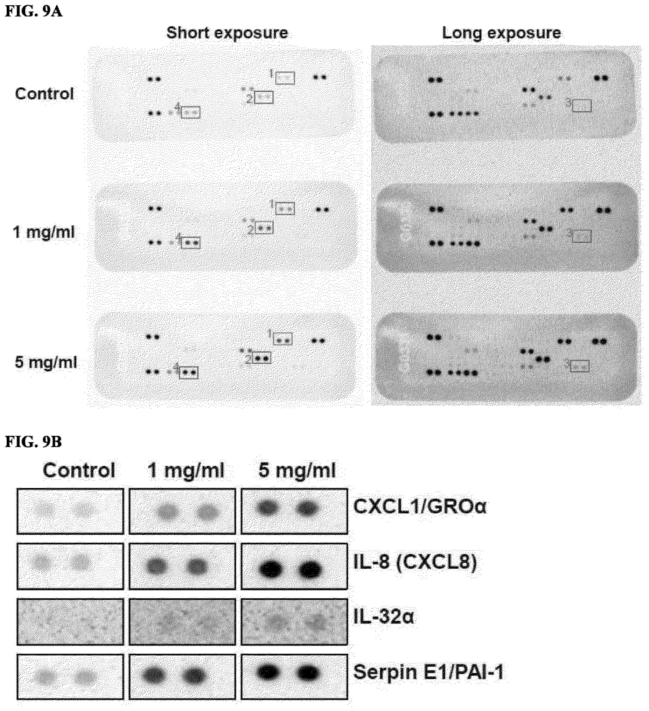

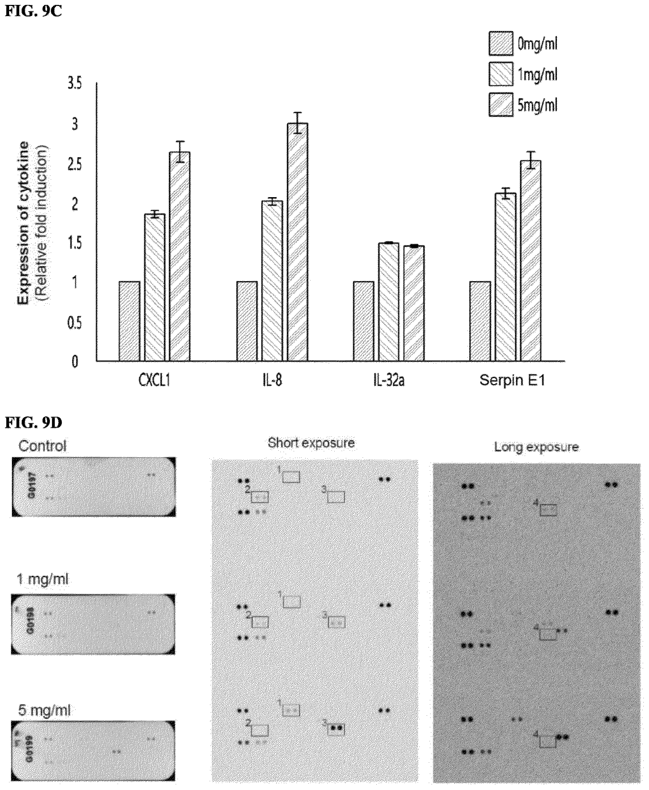

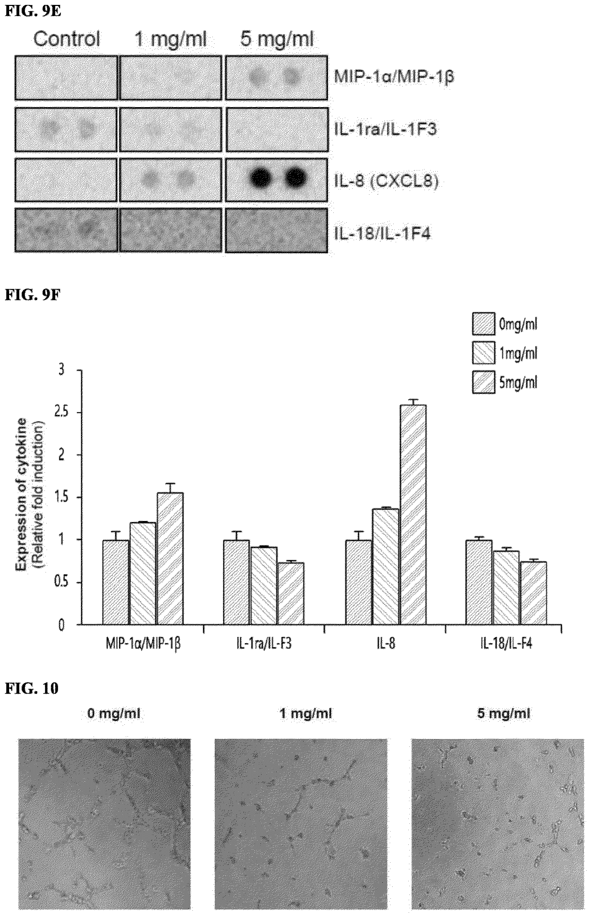

FIGS. 9A to 9F show the effect of the mixed extract of the present invention on cytokine production.
FIG. 10 shows the effect of the mixed extract of the present invention on angiogenesis.

### [Modes of the Invention]

### <Preparation Examples>

For the preparation of the mixed extract of the present invention, medicinal plants produced and distributed in Republic of Korea were used. Specifically, the medicine was mixed in a ratio of 40 to 150 parts by weight of *Prunella vulgaris* (spike), 30 to 120 parts by weight of *Akebia quinata* (fruit), 20 to 80 parts by weight of *Curcuma zedoaria* (rhizome), and 20 to 80 parts by weight of Curcumae Radix (*Curcumaphaeocaulis*) with respect to 100 parts by weight of *Hedyotis diffusa* (whole plant body). Thereafter, distilled water corresponding to about 10 times the medicine was added, and hot water was extracted at a temperature of 100°C in an extractor for 2 hours.

Then, the extracted mixed extract was filtered using a standard test sieve (150 µm, Retsch, Han, Germany), and after drying the extract for 6 hours at a temperature of -30°C under freeze-drying conditions, the extract was dried for 4 hours upon reaching a temperature of 30°C and concentrated until dried in a freeze dryer depending on the dry state. The freeze-dried mixed extract powder (50 mg) was dissolved in 1 ml of distilled water, filtered through a 0.22 µm disk filter, and stored at -20°C until use.

For comparison of anticancer efficacy, a single extract of *Hedyotis diffusa* (Comparative Example 1), *Prunella vulgaris* (Comparative Example 2), *Akebia quinata* (Comparative Example 3), *Curcuma zedoaria* (Comparative Example 4), and Curcumae Radix (Comparative Example 5) was prepared in the same manner as above. Then, an anticancer effect experiment was performed on the mixed extract of Preparation Example 1 (123 g of *Hedyotis diffusa,* 62 g of *Prunella vulgaris,* 92 g of *Akebia quinata,* 62 g of *Curcuma zedoaria,* and 62 g of Curcumae Radix). In addition, the mixed extracts of Preparation Examples 2 to 5 were prepared in the same manner as in Preparation Example 1, and anticancer-related experiments were performed. The composition is as described in Table 1 below (unit: g).

**[Table 1]**

| | *Hedyotis diffusa* | *Prunella vulgaris* | *Akebia quinata* | *Curcuma zedoaria* | Curcumae Radix |
|---|---|---|---|---|---|
| **Preparation Example 1** | 123 | 62 | 92 | 62 | 62 |
| **Preparation Example 2** | 92 | 92 | 92 | 62 | 62 |
| **Preparation Example 3** | 132 | 132 | 67 | 35 | 35 |
| **Preparation Example 4** | 88 | 131 | 89 | 46 | 46 |
| **Preparation Example 5** | 108 | 108 | 38 | 73 | 73 |

### <Example 1> Anticancer effects to the cancer cell lines

Cancer cell lines with different origins and characteristics: Huh7 (liver cancer), H460 (lung cancer), A549 (lung cancer), AGS (stomach cancer), HT29 (colorectal cancer), MDA-MB231 (breast cancer), A375 (melanoma), MiaPaCa-2 (pancreatic cancer), and SNU213 (pancreatic cancer) were used to measure the effects of the cancer cell proliferation inhibition and apoptosis of the mixed extract.

As cancer cells used in the experiment, H460 (ATCC, #HTB-177), A549 (ATCC, #CRM-CCL-185), AGS (ATCC, #CRL-1739), HT29 (ATCC, #HTB-38), MDA-MB231 (ATCC, #CRM-HTB-26), MiaPaCa-2 (ATCC, #CRM-CRL-1420), SNU213 (KCLB, #00213), and A375 (ATCC, #CRL-1619) were used.

All cancer cell lines were cultured in a medium containing 10% FBS (GIBCO, #GIB-26140-079) and 1% penicillin-streptomycin (GIBCO #GIB-15140-12). Specifically, DMEM-high glucose (Welgene #LM001-05) was used for A549, AGS, H529, MDA-MB231, MiaPaCa-2, and A375 among cancer cell lines, and RPMI-1640 (Welgene #LM011-03) was used for H460 and SNU213. The cancer cell line was subcultured once every 2-3 days and cultured in an incubator at 37°C.

In order to measure the cancer cell killing effects of the mixed extract of the present invention, first, liver cancer (Huh7), lung cancer (H460, and A549), stomach cancer (AGS), colorectal cancer (HT29), breast cancer (MDA-MB231), pancreatic cancer (MiaPaCa-2, and SNU213), melanoma (A375) cancer cell lines were seeded in 384-well plates at a density of 4,000 cells/40 µl/well or 2,000 cells/40 µl/well. Then, after overnight incubation for 24 hours, the extract of the present invention was treated at a concentration of 10 µl/well, followed by incubation at 37°C. After 48 hours, 40 µl/well of the medium was removed and 4% paraformaldehyde was added to be a 30 µl/well, followed by incubation at room temperature for 10 minutes. Thereafter, after washing with DPBS, nuclear staining was performed with Hoechst33342 at a concentration of 3 µl/ml at room temperature for 10 minutes. Finally, after washing twice with DPBS, images were acquired with Operetta.

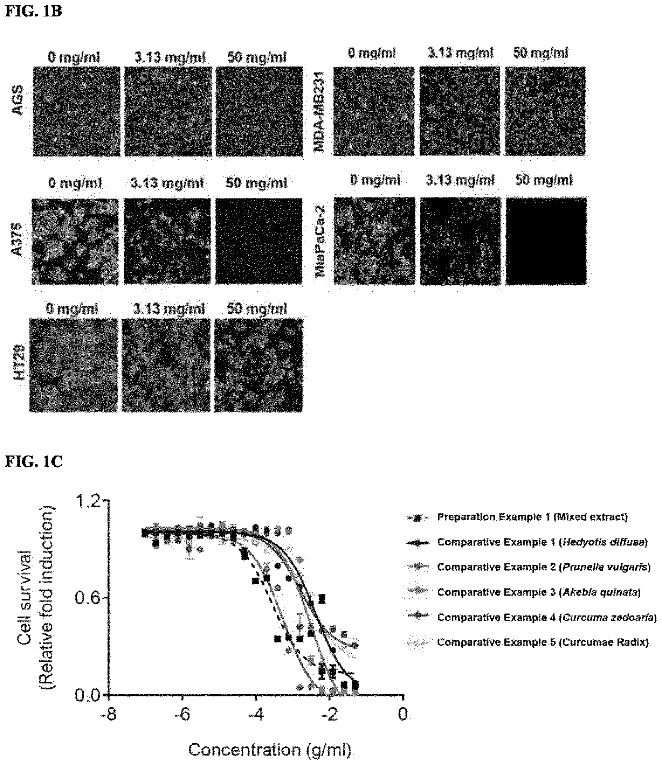

As a result, compared to the untreated control cells, the anticancer effect was remarkably observed when the mixed extracts at a concentration of 1-50 mg/ml were treated in various cancer cell lines. In other words, it was identified that the mixed extract of the present invention may effectively kill cancer cells in various carcinomas such as liver cancer, lung cancer, stomach cancer, colorectal cancer, breast cancer, pancreatic cancer, and melanoma (FIGS. 1A and 1B).

As a result of measuring the killing effect on carcinomas in a similar manner for the compositions of Preparation Examples 2 to 5 of the present invention, it was identified that cancer cells could be effectively killed as in Preparation Example 1. In addition, it was identified that the cancer cell killing effect of the mixed extract of the present invention was superior to that of each single medicinal extract configuring the mixed extract.

Specifically, the compositions of Comparative Examples 1 to 5 as a single extract were prepared through the same process as the mixed herbal extract, and the anticancer efficacy of Preparation Example 1, which is the mixed extract, was compared. Cell viability experiments were performed in the same manner as described above for the Huh7 (liver cancer) cell line. As a result, it was identified that the cancer cell killing effect was significantly improved in Preparation Example 1, which is directed to a mixed extract, compared to Comparative Examples 1 to 5, which are directed to a single extract. In particular, the anticancer effect (IC50 value) of the mixed herbal extract of the present invention was improved by 17.02 times compared to the single extract of *Hedyotis diffusa* (Comparative Example 1), and even compared with the extract of Curcumae Radix (Comparative Example 5), the anticancer effect (IC50 value) was improved by 9.66 times (Table 2 and FIG. 1C).

**[Table 2]**

| | **IC50 (mg/ml)** |
|---|---|
| **Pre. Ex. 1 (Mixed extract)** | 0.277 |
| **Comp. Ex. 1 (*Hedyotis diffusa*)** | 4.715 |
| **Comp. Ex.2(*Prunella vulgaris*)** | 0.547 |
| **Comp. Ex. 3 (*Akebia quinata*)** | 3.106 |
| **Comp. Ex.4(*Curcuma zedoaria*)** | 1.654 |
| **Comp. Ex.5(Carcumae Radix)** | 2.675 |

### <Example 2> Effect of Mixed Extract on Cancer-Related Protein Expression

In order to evaluate the effect of the mixed extract of the present invention on cancer-related mechanisms, the effect on the expression of cancer-related proteins was measured. First, in order to observe the expression patterns of cancer-related genes PARP/Cleaved PARP, Histone H3, UBE2C, and PLK1, cleaved caspase-3 (abcam, #ab2302) PARP & cleaved PARP (Cell signaling, #9532S), p-Histone H3 Ser10 (Cell signaling #53348S), UBE2C (cell signaling #4234S), PLK1 (Cell signaling #4513S), Apolipoprotein A1 (abeam, #ab7613), Apolipoprotein B (Novusbio #NBP2-37598), Apolipoprotein E (abeam, # ab1906) and β-actin (Sigma-Aldrich, #A5441) were prepared from each source.

For the experiment, Huh7, Hep3B, and SNU475 cells were seeded in 100 mm² dishes at 70% confluence. On the next day, after treatment with the extract of the present invention, it was cultured in an incubator at 37°C for 48 hours (concentration 15 times: dilution 5 times). Then, after harvesting the cells, the pellet was sampled with an e-tube, and protein extraction was performed according to the protocol. Then, the target protein change was identified by Western blotting (FIGS. 2A and 2B).

Then, in order to observe the expression pattern of SNAIL, a cancer-related gene, N-Cadherin (abeam, #ab76057), snail (Cell signaling, #3879S), a-SMA (abeam, #ab5694), and β-actin (Sigma-Aldrich, #A5441) were prepared from each source.

For the experiment, the Huh7 liver cancer cell line was seeded in 100-mm dishes at a density of 600,000 cells/6 ml/well. Then, after overnight incubation in an incubator at 37°C for 16 hours, the extracts of the present invention were treated at concentrations of 0, 1, 5, and 10 mg/ml. Thereafter, the cells were harvested after culturing for 48 hours in an incubator at 37°C, and then the pellet was sampled with an e-tube. Then, the change in the target protein level after protein extraction according to the protocol was identified through Western blotting.

As a result, it was identified that the mixed extract of the present invention increased the expression of proteins (cleaved Caspase-3 and PARP) involved in apoptosis of cancer cells (FIG. 2A), and inhibited the expression of cancer cell proliferation-related proteins (p-Histone H3, UBE2C, PLK1) (FIG. 2B). In addition, it was further identified that the mixed extract of the present invention inhibited the expression of proteins (N-cadherin, α-SMA, Snail) involved in the inhibition of cancer metastasis (FIG. 2C).

### <Example 3> Effect of Mixed Extract Used in Combination with Anticancer Drug

The effect of the mixed extract of the present invention on inhibition of cancer cell proliferation when used in combination with a conventional anticancer drug was measured. As a combined anticancer drug, sorafenib, known as a standard anticancer therapeutic agent for liver cancer cells, was used.

The cell viability measurement according to the use of sorafenib in combination was performed as follows. First, the Huh7 cell line was seeded in a 384-well plate at a density of about 2,000 cells/40 µl/well, and after overnight incubation for 24 hours, 0, 1, and 3 µM of sorafenib and 0, 1, and 5 mg/ml of the extract of the present invention were treated at 10 µl/well in combination, and then incubated in an incubator at 37°C. After 48 hours, 40 µl/well of the medium was removed, and 4% paraformaldehyde was added to be a 30 µl/well, followed by incubation at room temperature for 10 minutes. After washing twice with DPBS, images were acquired with Operetta. The cell viability was identified by counting the number of cells using Operetta software.

As a result, it was identified that the mixed extract of the present invention had an excellent anticancer effect when used in combination with sorafenib (FIG. 3).

### <Example 4> Effect of Mixed Extract on Anticancer Drug-Resistant Cancer

For cancer resistant to conventional anticancer drugs, in order to identify whether the anticancer effect is shown when the mixed extract of the present invention is treated, the proliferation inhibitory effect on the SNU475 cell line, which is known to exhibit resistance to the anticancer drug sorafenib, was compared with the same carcinoma cell lines, Huh7 and Hep3B. As shown in FIG. 4A, when sorafenib was treated in Huh7 and Hep3B cell lines among cancer cells, it was identified that the anticancer effect was exhibited in the 0, 1, and 3 µM range, whereas the SNU475 cell line exhibited resistance to the anticancer effect. In addition, the SNU475 cell line is known to exhibit strong resistance to the anticancer drug regorafenib in addition to sorafenib.

For cell viability measurements on cancer cell lines, three hepatocellular carcinoma cell lines (Huh-7, Hep3B, and SNU475) were seeded in a 384-well plate at a density of 2,000 cells/40 µl/well. After incubation for 16 hours, the extract of the present invention was treated at a concentration of 10 µl/well, followed by incubation at 37°C for 48 hours. After 48 hours, 40 µl/well of the medium was removed and 4% paraformaldehyde was added to be a 30 µl/well, followed by incubation at room temperature for 10 minutes. Thereafter, after washing with DPBS, nuclear staining was performed with Hoechst33342 at a concentration of 3 µl/ml at room temperature for 10 minutes. After washing twice with DPBS, images were acquired with Operetta. The cell viability was identified by counting the number of cells using Operetta software.

As a result, it was identified that the mixed extract of the present invention had excellent anticancer effects also in SNU475, which is the cancer resistant to anticancer drugs such as sorafenib or regorafenib, like Huh-7 and Hep3B, which do not have resistance to anticancer drugs (FIG. 4B).

In addition, in order to examine whether the mixed extract of the present invention has a metastasis inhibitory effect in anticancer drug-resistant cancer, the metastasis inhibitory effect was identified in the same manner as in Example 5, which will be described later. As a result, it was identified that, in addition to the cancer cell killing effect, the metastasis inhibitory effect was also exhibited in SNU475, an anticancer drug-resistant cancer (FIG. 4C).

### <Example 5> Cancer Metastasis Inhibitory Effect of Mixed Extract

In order to identify that the mixed extract of the present invention may inhibit metastasis of cancer cells, a migration assay for cancer metastasis measurement was performed.

For migration assay experiments, the Huh7 cell line was seeded in 12-well plates at a density of 300,000 cells/2 ml/well. Then, after overnight incubation in an incubator at 37°C for 16 hours, a wound was made by scratching the center of the well with a 200 µl tip. After washing with DPBS, the extract of the present invention was treated with concentrations of 0, 1, 5, and 10 mg/ml, and wound images were captured. Then, after 24 hours of incubation in an incubator at 37°C, the degree of cell migration was checked and wound images were captured.

As a result, the cancer metastasis inhibitory effect of the mixed extract of the present invention was identified (FIG. 5).

### <Example 6> Effect of Mixed Extract on Cancer Cell Cycle

In order to evaluate the effect of the mixed extract of the present invention on the cell cycle of cancer, Sub-G1 analysis was performed as follows.

For the experiment, Huh7 and SNU475 cell lines were seeded at a density of about 70-80% in 100 mm dishes. Then, after overnight incubation in an incubator at 37°C, the extracts of the present invention were treated at concentrations of 0, 1, and 5 mg/ml, followed by incubation in an incubator at 37°C. After 6, 12, 24, and 48 hours of treatment with the extract of the present invention, all of the medium and cells were harvested, washed with PBC, fixed with 70% ethanol, and stored at -20°C. PI/RNase staining solution was added to each sample, and FACS analysis was performed. The pattern of cell division was identified by analyzing the degree of PI staining through FACS.

As a result, it was identified that the mixed extract of the present invention inhibited cancer cell proliferation by increasing the Sub-G1 cell cycle of cancer cells (FIGS. 6A to 6C). In addition, it was identified that the mixed extract of the present invention increased the Sub-G1 cell cycle even in anticancer drug (sorafenib)-resistant cancer (FIGS. 6D to 6F).

### <Example 7> Effect of Mixed Extract on Accumulation of Reactive Oxygen Species in Cancer Cells

In order to identify that the mixed extract of the present invention contributes to the cancer cell killing effect by increasing the production of reactive oxygen species in cancer cells, the ROS measurement experiment was performed as follows.

For experiments, Huh7, Hep3B cell lines were seeded in 384-well plates at a density of 5,000 cells/40 µl/well. Then, after overnight incubation in an incubator at 37°C, the extracts of the present invention were treated at concentrations of 0, 1, 5, and 10 mg/ml, and then incubated in an incubator at 37°C. After 1, 3, and 6 hours of treatment with the extract of the present invention, the medium was removed and 1,000x H2DCFDA (ROS detection reagent) was diluted at a concentration of 1x in a culture medium, and then was incubated with 3 µl/ml Hoechst in an incubator at 37°C for 10 minutes. Thereafter, the medium was removed again and washed quickly with DPBS, and images were acquired with Operetta. The degree of ROS production was identified by analyzing the cell number and ROS intensity using Operetta software.

As a result, it was identified that the mixed extract of the present invention inhibited cancer cell proliferation by increasing the accumulation of reactive oxygen species in cancer cells (FIGS. 7A to 7D).

### <Example 8> Effects of Mixed Extract on Immune Cells

In order to identify whether the mixed extract of the present invention contributes to the anticancer effect through the proliferation of immune cells, the following experiment was performed using M1 macrophages.

For the experiment, bone-marrow-derived macrophage (BMDM) cells acquired from C57BL/6J mice were treated with M-CSF 50 ng/ml in a Petri dish and cultured for 7 days, and then BMDMs treated with 5 ng/ml M-CSF (24h) were seeded in 384-well plates at a density of 2.5 × 10³ cells/well. Then, PBS (M0 macrophage culture condition), LPS 50 ng/ml, IFN-y 20 ng/ml (M1 macrophage culture condition), IL-4, IL-13 50 ng/ml (M2 macrophage culture condition), and 1, 5, and 10 mg/ml of the extract of the present invention were treated in a 384-well plate seeded with BMDM, followed by incubation for 24 hours. Thereafter, the medium was removed, washed with DPBS, and stained with WGA488 (for total cell morphology analysis) and Hoechst33342 (for nuclear morphology and cytotoxicity analysis) at room temperature for 10 minutes. After rapid washing with DPBS, images were acquired with Operetta. The degree of macrophage polarization was analyzed through cell number and cell morphology analysis using Operetta software.

As a result, it was identified that the mixed extract of the present invention had an effect of increasing M1 macrophages, which are immune cells (FIGS. 8A and 8B).

### <Example 9> Effect of Mixed Extract on Cytokine Production

In order to confirm whether the mixed extract of the present invention contributes to the anticancer effect through immune activation by increasing cytokine secretion of cancer cells or immune cells, the following experiment was performed.

For proteome profiler human cytokine array experiments on Huh7 cells, Huh7 cells were seeded in a 100 mm dish at a density of 700,000 cells/dish. Then, after overnight incubation for 16 hours at 37°C, the extract of the present invention was treated with each cell at a concentration of 0, 1, and 5 mg/ml and incubated at 37°C. After 48 hours, the harvested cells were lysed with RIPA-buffer to quantify the protein. 200 µg of protein was obtained and a mixture mixed with Human cytokine array detection antibody cocktail was incubated overnight at 4°C on a rocking platform shaker with a membrane. After 16 hours, the membrane was washed twice for 10 minutes each, and the membrane was placed in an array buffer diluted with Streptavidin-HRP and incubated for 30 minutes. Thereafter, the membrane was washed twice for 10 minutes each, and then the Chemi Reagent mixture was treated on the membrane, and an image was acquired using a Luminograph Gel image.

As a result, it was identified that the mixed extract of the present invention promoted cytokine production in cancer cells (FIGS. 9A to 9C).

For proteome profiler human cytokine array experiments on THP-1, the THP-1 cell line was first obtained from ATCC (cat no. TIB-202). THP-1 cells were seeded in a 100 mm dish at a density of 1,000,000 cells/dish. Then, after overnight incubation for 16 hours at 37°C, the extract of the present invention was treated with each cell at a concentration of 0, 1, and 5 mg/ml and incubated at 37°C. After 48 hours, the harvested cells were lysed with RIPA-buffer to quantify the protein. 200 µg of protein was obtained and a mixture mixed with Human cytokine array detection antibody cocktail was incubated overnight at 4°C on a rocking platform shaker with a membrane. After 16 hours, the membrane was washed twice for 10 minutes each, and the membrane was placed in an array buffer diluted with Streptavidin-HRP and incubated for 30 minutes. The membrane was washed twice for 10 minutes each, and then the Chemi Reagent mixture was treated on the membrane, and an image was acquired using a Luminograph Gel image.

As a result, it was identified that the mixed extract of the present invention promoted cytokine production in immune cells (FIGS. 9D to 9F).

### <Example 10> Effects of Mixed Extract on Angiogenesis

In order to identify whether the mixed extract of the present invention contributes to the anticancer effect through immune activation by increasing cytokine secretion of cancer cells or immune cells, the following experiment was performed.

The HUVEC cell line used in the experiment was obtained from Promocell (cat no. C-12208). First, 100 µl of growth factor-reduced Matrigel was dispensed in a 96-well plate, and then incubated for 1 hour at room temperature. Then, HUVEC cells cultured at 70-80% confluency were subjected to serum starvation for 3 hours. HUVEC cells were removed from the plate, resuspended in serum-free DMEM, and centrifuged for 3 minutes at 4,000 rpm for 200,000 cells. After discarding the supernatant, it was dissolved in 500 µl of the culture solution diluted with 0, 1, and 5 mg/ml of the extract of the present invention (so that the final concentration of FBS was 1%), and then 40,000 cells/well were seeded in a 96-well plate applied with Matrigel. Thereafter, after culturing for 4-6 hours in an incubator at 37°C, the degree of tube formation was checked using an optical microscope.

As a result, it was identified that the mixed extract of the present invention effectively blocked angiogenesis required for cancer cell proliferation (FIG. 10).

## Claims

1. An anticancer pharmaceutical composition comprising an extract of *Hedyotis diffusa, Prunella vulgaris, Akebia quinata, Curcuma zedoaria,* and Curcumae Radix.

2. The composition of claim 1, wherein the extract is prepared by extracting 40 to 150 parts by weight of *Prunella vulgaris,* 30 to 120 parts by weight of *Akebia quinata,* 20 to 80 parts by weight of *Curcuma zedoaria,* and 20 to 80 parts by weight of Curcumae Radix with respect to 100 parts by weight of *Hedyotis diffusa.*

3. The composition of claim 2, wherein the extract is prepared by extracting *Hedyotis diffusa, Prunella vulgaris, Akebia quinata, Curcuma zedoaria,* and Curcumae Radix with water, a C1-C4 alcohol, or a mixed solvent thereof.

4. The composition of claim 3, wherein the extract is prepared by hot water extraction, ultrasonic extraction, room temperature extraction, cold-immersion extraction, reflux cooling extraction, or vapor extraction.

5. The composition of claim 1, wherein the extract has cancer cell killing and cancer cell metastasis inhibitory activity.

6. The composition of claim 1, wherein the cancer is selected from the group consisting of liver cancer, lung cancer, pancreatic cancer, stomach cancer, colorectal cancer, breast cancer, and melanoma.

7. An anticancer health functional food comprising an extract of *Hedyotis diffusa, Prunella vulgaris, Akebia quinata, Curcuma zedoaria,* and Curcumae Radix.
